# EUROPÄISCHE PATENTSCHRIFT

(11) **EP 0 935 967 B1**
(45) Veröffentlichungstag und Bekanntmachung des Hinweises auf die Patenterteilung: **04.05.2005**
(21) Anmeldenummer: 98119299.0
(22) Anmeldetag: 13.10.1998
(51) Int. Cl.: A61M 1/28, A61J 1/05, A61K 9/08, A61K 33/10, A61K 33/06, A61K 31/70

(54) **Lösung für die Peritonealdialyse**
Peritoneal dialysis solution
Solution pour dialyse péritonéale

(30) Priorität: 31.10.1997 DE 19748290
(43) Veröffentlichungstag der Anmeldung: 18.08.1999
(73) Patentinhaber: Fresenius Medical Care Deutschland GmbH, 61350 Bad Homburg v.d.H. (DE)
(72) Erfinder: Knerr, Thomas, Dr., 66606 St. Wendel (DE)
(74) Vertreter: Laufhütte, Dieter, Dr.-Ing.

(56) Entgegenhaltungen:
- EP-A- 0 399 549
- EP-A- 0 821 951
- WO-A-91/08008
- WO-A-93/09820
- WO-A-96/01118
- WO-A-97/06810
- PATENT ABSTRACTS OF JAPAN vol. 1997, no. 07, 31. Juli 1997 (1997-07-31) & JP 09 087182 A (TERUMO CORP), 31. März 1997 (1997-03-31)

## Beschreibung

Die Erfindung betrifft eine Lösung für die Peritonealdialyse oder zur Infusion nach dem Oberbegriff des Anspruchs 1.

In einer Peritonealdialyselösung sind grundsätzlich drei funktionelle Bestandteile enthalten. Zunächst sinc Elektrolyte enthalten, wobei es sich hier um Calcium-, Natrium- und Magnesiumsalze, die in der Regel als Chloride eingesetzt werden, handelt. Als zweiter funktionaler Bestandteil ist ein Puffer vorgesehen. Das verträglichste Puffersystem besteht aus Bicarbonat, das im basischen Bereich mit Carbonat und im sauren Bereich mit CO₂ im Gleichgewicht steht. Einsetzbar als Puffer wären auch Stoffe, die in einem pH-Wert von etwa 7, also einem physiologischen pH-Wertpuffern. Bevorzugt sind Stoffe, die im Körper leicht zu Bicarbonat metabolisiert werden können, wie Lactat, Pyruvat oder ähnliche Stoffe. Der dritte funktionelle Bestandteil besteht aus einem Osmotikum. Hier wird häufig Glucose verwendet, die bei relativ niedriger Konzentration eine hohe Osmolarität aufweist und gut verträglich ist. Für den Einsatz von Glucose spricht insbesondere der Preisvorteil gegenüber anderen möglicherweise als Osmotikum einsetzbaren Stoffen.

Eine Lösung für die Peritonealdialyse nach dem Oberbegriff des Anspruchs 1 ist beispielsweise aus der WO 96/01118 bekannt. Dort wird Bicarbonat in physiologischen Mengen von 20-30 mEq/l zusammen mit einer schwachen Säure in einer Menge von 10-20 mEq/l eingesetzt. Bei Verwendung einer derartigen Peritonealdialyselösung ist es notwendig, daß das enthaltene Bicarbonat und das enthaltene Calcium getrennt gelagert werden, da sich bei einer gemeinsamen Lagerung sehr leicht ein unlöslicher Calciumcarbonat-Niederschlag bildet. Im sauren Bereich kann zwar dieser Niederschlag vermieden werden, da sich Bicarbonat weiter im Gleichgewicht mit Kohlensäure und damit mit CO₂ befindet, so daß weniger Carbonat vorliegt. Hieraus entsteht aber der Nachteil, daß ein relativ hoher CO₂ Partialdruck entsteht. Dieser hohe CO₂ Partialdruck wiederum fordert eine Beutelfolie mit einer wirksamen CO₂ Barriere, so daß besonders angepaßte Beutel mit einer entsprechenden CO₂ Sperrschicht zum Einsatz kommen können.

In der EP 0 076 355 A ist zur Vermeidung dieser Handling-Probleme von Bicarbonat enthaltender Lösung das Bicarbonat beispielsweise durch Lactat als Puffer ersetzt. Die Verwendung von Lactat führt aber bei der Hitzesterilisation zu einem anderen Problem, da Lactat und die in der Lösung auch vorhandene Glucose zu Acetaldehyd reagieren. Acetaldehyd schädigt aber die Peritonealwände.

Die Hitzesterilisation führt darüber hinaus auch dazu, daß die Glucose karamelisiert, isomerisiert oder zu Produkten abgebaut wird, die im Körper irreversibel mit Proteinen weiterreagieren können. Dies und andere Probleme führten dazu, daß in der EP 0 076 355 A Glucose zum Beispiel durch Glucosepolymere (wie Icodextrin), Peptide oder Proteine wie Albumin ersetzt worden ist. Dieser Glucoseersatz führt aber zu einer erheblichen Verteuerung des Produkts. Darüber hinaus wurden bei Einsatz dieser Ersatzmittel physiologische Reaktionen, wie beispielsweise Immunreaktionen, beobachtet. Schließlich konnten ähnliche Probleme wie bei der Glucose auch bei den Glucosepolymeren während der Hitzesterilisation festgestellt werden, so daß nach anderen Wegen gesucht werden muß, um den Glucoseabbau zu vermeiden.

Hier lehrt die WO 93/09820 für ein entsprechendes System zunächst eine getrennte Lagerung und zum anderen eine kurze, aber dafür hohe Erhitzung zur Sterilisation. Bei der getrennten Lagerung wird die Glucose zu einem großen Teil in einem gesonderten Behältnis gelagert, um Reaktionen mit anderen Bestandteilen, wie beispielsweise Lactat, zu vermeiden. Die Glucoselösung kann nach dieser Lehre kurz und sehr hoch erhitzt werden, während die Lösung mit den anderen Bestandteilen den üblichen Sterilisationsbedingungen problemlos unterzogen werden kann.

Eine andere Möglichkeit, die Probleme, die hinsichtlich der EP 0 076 355 A geschildert worden sind, zu lösen, ist in der WO 91/08008 beschrieben, wo eine Sterilisation bei sehr niedrigen pH-Werten unter 3 durchgeführt werden kann. Hier ergibt sich wiederum das Problem, daß bei diesen niedrigen pH-Werten eine schwache Säure, wie beispielsweise Milchsäure oder ihr Salz, das Lactat als schwache Base zur Pufferung nicht ausreichen, um in einer Endlösung einen physiologischen pH-Wert um 7,2 bis 7,4 zu erreichen.

Hieraus ergibt sich die Aufgabe, eine Lösung für die Peritonealdialyse oder zur Infusion bereitzustellen, die einerseits eine gute Lagerbarkeit und ein einfaches und sicheres Handling und andererseits eine gute Biokompatibilität gewährleistet.

Ausgehend von einer Lösung nach dem Oberbegriff des Anspruchs 1 wird diese Aufgabe durch die Kombination mit den Merkmalen des kennzeichnenden Teils des Anspruchs 1 gelöst. Demnach wird die erste Einzellösung mit einer physiologisch verträglichen Säure auf einen pH-Wert unter 3,2 angesäuert. Die zweite Einzellösung weist einen Gehalt an Bicarbonat auf, der 10 mmol/l nicht überschreitet.

Erfindungsgemäß wird hier ein Puffer aus Lactat und Bicarbonat bereitgestellt. Aufgrund des Bicarbonatanteils kann in einer Endlösung ein physiologischer pH-Wert um 7,2 bis 7,4 erreicht werden. Wesentlich ist es, daß der Gehalt von Bicarbonat unter 10 mmol/l liegt, da hierdurch überraschenderweise der Druck des CO₂ innerhalb des Aufbewahrungsbeutels so niedrig ist, daß die Barriere einer normalen Polyolefinfolie bzw. einer normalen PVC-Folie ausreicht, um die Bicarbonatkonzentration konstant zu halten. Die schwierigen Handlingprobleme der üblicherweise Bicarbonat enthaltenden Lösung bzw. die Erfordernisse einer CO₂-Barriere-Folie kann dadurch umgangen werden. Um den Bicarbonatgehalt so gering zu halten, besteht der Hauptpuffer aus einem Salz einer physiologischen schwachen Säure, vorzugsweise Lactat, dessen Pufferkapazität in diesem kombinierten Puffer von dem Bicarbonat lediglich gestützt wird. Während Lactat und Bicarbonat in einer Beutelkammer gelagert werden können, können Glucose und die Elektrolyte - und mit den Elektrolyten zusammen das Calciumion - in einer zweiten Kammer auf einen pH unter 3,2 angesäuert und sterilisiert werden. Nach der Sterilisation können die Inhalte beider Kammern vereinigt, gemischt und dem Patienten verabreicht werden.

Die erfindungsgemäße Lösung weist einen verträglichen pH-Wert und weniger Glucoseabbauprodukte auf. Dadurch erreicht sie eine hohe Biokompatibilität. Besonders vorteilhaft ist, daß mit der erfindungsgemäßen Lösung aufgrund der Verringerung des Glucoseabbaus auch die Einlaufschmerzen bei Dialysepatienten vermieden werden können.

Vorteilhafte Ausgestaltungen der Erfindung ergeben sich aus den Unteransprüchen 2 bis 11.

Nach einer besonders vorteilhaften Ausführungsform weist die erste Einzellösung, die die Calciumionen, weitere Elektrolytsalze und die Glucose enthält, einen pH-Wert von 2,8 bis 3,2, vorzugsweise einen pH-Wert von 3, auf. Durch die Ansäuerung dieser Einzellösung, die die Glucose enthält, wird eine deutliche Reduktion des Glucoseabbaus bei der Sterilisation erhalten. In der zweiten Einzellösung, die Bicarbonat und das Salz einer schwachen Säure stellt sich, gehört ein pH-Wert von 8 bis 8,5. Bei Mischung der Einzellösungen im Verhältnis von 1 zu 1 ergibt sich bei der fertigen Lösung ein pH-Wert von 7,2 bis 7,4.

In der ersten Einzellösung können neben Calciumionen und Glucose als weitere Elektrolyten Natriumionen, Magnesiumionen, H⁺-Überschußionen und Chloridionen enthalten sein. In der zweiten Einzellösung können neben Natriumionen und Lactationen Hydrogencarbonationen enthalten sein.

Vorteilhaft ist die physiologisch verträgliche Säure zur Ansäuerung auf einen pH-Wert unter 3,2 Salzsäure.

Eine besonders verträgliche Aufteilung der Lösung auf die zwei Einzellösungen gibt sich wie folgt:
1. Einzellösung:

| | |
|---|---|
| Natrium [mmol/l] | 180-200, vorzugsweise 193 |
| Calcium [mmol/l] | 2-4, vorzugsweise 2,5 oder 3,5 |
| Magnesium [mmol/l] | 0,8-1,2, vorzugsweise 1,0 |
| H⁺-Überschuß [mmol/l] | 0,9-1,1, vorzugsweise 1,0 |
| Chlorid [mmol/l] | 197-210, vorzugsweise 203 |
| Glucose [mmol/l] | 100-500, vorzugsweise 166,5, 252 oder 472 |

2. Einzellösung:

| | |
|---|---|
| Natrium [mmol/l] | 70-80, vorzugsweise 75 |
| Lactat [mmol/l] | 65-75, vorzugsweise 70 |
| Hydrogencarbonat [mmol/l] | 4-6, vorzugsweise 5,0. |

Ein besonders gutes Handling der beiden Lösungen ergibt sich bei Verwendung eines Doppelkammerbeutels, der beispielsweise aus einem üblichen Polyolefin bestehen kann nach Anspruch 11. Demnach besteht dieser Kunststoffbeutel aus einer ersten Kammer für die erste Lösung und einer zweiten Kammer für die zweite Lösung, die benachbart zueinander angeordnet sind, wobei beide Kammern durch eine Schweißnaht voneinander abgetrennt sind, die derart dimensioniert ist, daß sie sich bei Druck auf eine der flüssigkeitsgefüllten Kammern öffnet, so daß der Inhalt der beiden Kammern miteinander vermischbar ist.

Grundsätzlich waren zwar bereits Doppelkammerbeutel im Stand der Technik bekannt. Diese besaßen aber als trennendes Teil zwischen den beiden Kammern jeweils ein Spritzgußteil, das durch Abbrechen geöffnet wurde. In der Regel sind hier aber dann nur Durchflußöffnungen von weniger als 10 mm geöffnet worden. Da nun zum Mischen der Lösungen, eine Lösung von der einen Kammer in die andere und wieder zurück gedrückt werden muß, ergab sich eine zeitaufwendige Mischprozedur, die die Akzeptanz des Doppelkammerbeutels bei dem Patienten herabsetzte. Diese Nachteile sind durch den neuen Doppelkammerbeutel ausgeräumt worden.

Im folgenden wird ein Beispiel für die Herstellung einer erfindungsgemäßen Lösung gegeben:

Zur Herstellung der ersten Einzellösung werden in 973 ml Wasser für Injektionszwecke 11,279 g Natriumchlorid, 0,5145 Calciumchlorid x 2H₂O, 0,2033 g Magnesiumchlorid x 6H₂O, 33 g Glucose Monohydrat für Injektionszwecke und 0,130 ml 25%ige Salzsäure unter Rühren in Lösung gebracht. Eine notwendige Korrektur des pH-Wertes kann durch Hinzufügen bzw. Weglassen von 25%iger Salzsäure oder Natriumhydroxid erfolgen. Die so exakt auf den gewünschten pH-Wert eingestellte Lösung wird durch Membranvorfilter und anschließend durch Membransterilfilter in einen Kühltank filtriert. Nach Ansatzkontrolle und Freigabe der Lösung wird diese in einen Doppelkammermehrschichtfolienbeutel gefüllt und mit Konnektoren verschlossen. Der trockene Beutel wird anschließend in einen Umbeutel umverpackt und dann hitzesterilisiert bei 121°C.

Bei der zweiten Einzellösung wird in 986 ml Wasser für Injektionszwecke, das auf 12 bis 14°C gekühlt wird 15,69 g Natriumlactat als 50%ige Lösung und 0,420 g Natriumhydrogencarbonat unter langsamem Rühren gelöst. Die Temperatur der Lösung soll während der Ansatz- und Lagerzeit 20°C nicht überschreiten. Die Lösung wird anschließend durch Membranvorfilter und Membransterilfilter in einen Kühltank filtriert. Nach Durchführung der Ansatzkontrolle und Freigabe der Lösung wird diese in den Doppelkammerbeutel gefüllt und mit Konnektoren verschlossen. Der trockene Beutel wird mit einem Umbeutel umverpackt. Anschließend wird bei 121°C sterilisiert.

Für den Einsatz werden die beiden Einzellösungen im Verhältnis 1 zu 1 gemischt.

## Patentansprüche

1. Lösung für die Peritonealdialyse oder zur Infusion bestehend aus zwei Einzellösungen, die nach einer Hitzesterilisation zusammengeführt und verabreicht werden, wobei die erste Einzellösung Calciumionen, weitere Elektrolytsalze und Glucose in osmotisch wirksamer Konzentration enthält, und wobei die zweite Einzellösung Bicarbonat und das Salz einer schwachen Säure mit pka < 5 enthält,
**dadurch gekennzeichnet,**
**daß** die erste Einzellösung mit einer physiologisch verträglichen Säure auf einen pH-Wert unter 3,2 angesäuert ist und daß die zweite Einzellösung einen Gehalt an Bicarbonat aufweist, der 10 mmol/l nicht überschreitet.

2. Lösung nach Anspruch 1, **dadurch gekennzeichnet, daß** die erste Einzellösung, die die Calciumionen, weitere Elektrolytsalze und Glucose enthält, einen pH-Wert von 2,8 bis 3,2, vorzugsweise einen pH-Wert von 3 aufweist, und daß die zweite Einzellösung, die Bicarbonat und eine schwache Säure enthält, einen pH-Wert von 8 bis 8,5 enthält, so daß sich bei einer Mischung der Einzellösungen im Verhältnis von 1 zu 1 bei der fertigen Lösung ein pH-Wert von ca. 7,2 bis 7,4 einstellt.

3. Lösung nach Anspruch 1 oder 2, **dadurch gekennzeichnet, daß** in der ersten Einzellösung neben Calciumionen und Glucose Natriumionen, Magnesiumionen, H⁺-Überschußionen und Chloridionen enthalten sind.

4. Lösung nach einem der Ansprüche 1 bis 3, **dadurch gekennzeichnet, daß** in der zweiten Einzellösung neben Natriumionen, Salze schwacher Säuren z.B. Pyruvationen, Lactationen und Hydrocarbonationen enthalten sind.

5. Lösung nach einem der Ansprüche 1 bis 4, **dadurch gekennzeichnet, daß** die physiologisch verträgliche Säure zur Ansäurung auf einen pH-Wert unter 3,2 Salzsäure ist.

6. Lösung nach einem der Ansprüche 1 bis 5, **dadurch gekennzeichnet, daß** die erste Einzellösung folgende Bestandteile umfaßt:
| | |
|---|---|
| Natrium [mmol/l] | 180-200 |
| Calcium [mmol/l] | 2-4 |
| Magnesium [mmol/l] | 0,8-1,2 |
| H⁺-Überschuß [mmol/l] | 0,9-1,1 |
| Chlorid [mmol/l] | 197-210 |
| Glucose [mmol/l] | 100-500. |

7. Lösung nach einem der Ansprüche 1 bis 6, **dadurch gekennzeichnet, daß** die zweite Einzellösung folgende Bestandteile umfaßt:
| | |
|---|---|
| Natrium [mmol/l] | 70-80 |
| Lactat [mmol/l] | 65-75 |
| Hydrogencarbonat [mmol/l] | 4-6 |

8. Lösung nach Anspruch 6, **dadurch gekennzeichnet, daß** die erste Einzellösung folgende Bestandteile umfaßt:
| | |
|---|---|
| Natrium [mmol/l] | 193 |
| Calcium [mmol/l] | 2,5 oder 3,5 |
| Magnesium [mmol/l] | 1,0 |
| H⁺-Überschuß [mmol/l] | 1,0 |
| Chlorid [mmol/l] | 203 |
| Glucose [mmol/l] | 166,5, 252 oder 472 |

9. Lösung nach Anspruch 7, **dadurch gekennzeichnet, daß** die zweite Einzellösung folgende Bestandteile umfaßt:
| | |
|---|---|
| Natrium [mmol/l] | 75 |
| Lactat [mmol/l] | 70 |
| Hydrogencarbonat [mmol/l] | 5,0 |

10. Lösung nach einem der Ansprüche 1 bis 9, **dadurch gekennzeichnet, daß** die beiden Einzellösungen in einem Doppelkammerbeutel getrennt lagerbar sind.

11. Doppelkammerbeutel enthaltend eine Lösung nach einem der Ansprüche 1 bis 10, **dadurch gekennzeichnet, daß** er aus einem Kunststoffbeutel besteht, in dem eine erste Kammer mit der ersten Lösung und eine zweite Kammer mit der zweiten Lösung benachbart zueinander angeordnet sind, wobei beide Kammern durch eine Schweißnaht voneinander abgetrennt sind, die derart dimensioniert ist, daß sie sich bei Druck auf eine der flüssigkeitsgefüllten Kammern öffnet, so daß der Inhalt der beiden Kammern miteinander vermischbar ist.

## Claims

1. A solution for peritoneal dialysis or for infusion comprising two individual solutions which after a heat sterilisation operation are brought together and administered, wherein the first individual solution contains calcium ions, further electrolytic salts and glucose in an osmotically effective concentration, and wherein the second individual solution contains bicarbonate and the salt of a weak acid with pka < 5,
**characterised in that**
the first individual solution is acidified with a physiologically compatible acid to a pH-value of below 3.2 and that the second individual solution has a bicarbonate content which does not exceed 10 mmol/l.

2. A solution according to claim 1 **characterised in that** the first individual solution which contains the calcium ions, further electrolytic salts and glucose has a pH-value of 2.8 to 3.2, preferably a pH-value of 3, and the second individual solution which contains bicarbonate and a weak acid has a pH-value of 8 to 8.5 so that upon mixing of the individual solutions in a ratio of 1 to 1 the finished solution has a pH-value of about 7.2 to 7.4.

3. A solution according to claim 1 or claim 2 **characterised in that** besides calcium ions and glucose the first individual solution contains sodium ions, magnesium ions, H⁺excess ions and chloride ions.

4. A solution according to one of claims 1 to 3 **characterised in that** contained in the second individual solution besides sodium ions are salts of weak acids, for example pyruvate ions, lactate ions and hydrocarbonate ions.

5. A solution according to one of claims 1 to 4 **characterised in that** the physiologically compatible acid for acidification to a pH-value below 3.2 is hydrochloric acid.

6. A solution according to one of claims 1 to 5 **characterised in that** the first individual solution includes the following constituents:
| | |
|---|---|
| sodium [mmol/l] | 18-200 |
| calcium [mmol/l] | 2-4 |
| magnesium [mmol/l] | 0.8-1.2 |
| H⁺-excess [mmol/l] | 0.9-1.1 |
| chloride [mmol/l] | 197-210 |
| glucose [mmol/l] | 100-500. |

7. A solution according to one of claims 1 to 6 **characterised in that** the second individual solution includes the following constituents:
| | |
|---|---|
| sodium [mmol/l] | 70-80 |
| lactate [mmol/l] | 65-75 |
| hydrogen carbonate [mmol/l] | 4-6. |

8. A solution according to claim 6 **characterised in that** the first individual solution includes the following constituents:
| | |
|---|---|
| sodium [mmol/l] | 193 |
| calcium [mmol/l] | 2.5 or 3.5 |
| magnesium [mmol/l] | 1.0 |
| H⁺-excess [mmol/l] | 1.0 |
| chloride [mmol/l] | 203 |
| glucose [mmol/l] | 166.5, 252 or 472. |

9. A solution according to claim 7 **characterised in that** the second individual solution includes the following constituents:
| | |
|---|---|
| sodium [mmol/l] | 75 |
| lactate [mmol/l] | 70 |
| hydrogen carbonate [mmol/l] | 5.0. |

10. A solution according to one of claims 1 to 9 **characterised in that** the two individual solutions can be stored separately in a double-chamber bag.

11. A double-chamber bag containing a solution according to one of claims 1 to 10 **characterised in that** it comprises a plastic bag in which a first chamber with the first solution and a second chamber with the second solution are arranged in mutually adjacent relationship, wherein the two chambers are separated from each other by a weld seam which is so dimensioned that when pressure is applied to one of the liquid-filled chambers the weld seam opens so that the content of the two chambers can be mixed together.

## Revendications

1. Solution pour la dialyse péritonéale ou bien pour la perfusion se composant de deux solutions individuelles qui sont rassemblées après une stérilisation à la chaleur et sont distribuées, la première solution individuelle contenant des ions calcium, d'autres sels d'électrolyte et du glucose à une concentration osmotique efficace, et la seconde solution individuelle contenant du bicarbonate et le sel d'un acide faible avec pka<5,
**caractérisée en ce que**,
la première solution individuelle est acidifiée à un pH en dessous de 3,2 au moyen d'un premier acide physiologiquement compatible et **en ce que** la seconde solution individuelle présente une teneur en bicarbonate qui ne doit pas dépasser 10 mmoles/l.

2. Solution selon la revendication 1, **caractérisée en ce que** la première solution individuelle, qui contient les ions calcium, d'autres sels d'électrolytes et le glucose, présente un pH de 2,8 à 3,2, avantageusement un pH de 3, et **en ce que** la seconde solution individuelle, qui contient du bicarbonate et un acide faible, contient un pH de 8 à 8,5 donc, lors d'un mélange des solutions individuelles à un rapport de 1 à 1, dans la solution terminée, il règne un pH d'environ 7,2 à 7,4.

3. Solution selon la revendication 1 ou 2, **caractérisée en ce que** dans la première solution individuelle, outre les ions calcium et le glucose, des ions sodium, des ions magnésium, des ions H⁺ en excès et des ions chlorure sont contenus.

4. Solution selon l'une quelconque des revendications 1 à 3, **caractérisée en ce que** dans la seconde solution individuelle, outre les ions sodium, les sels, des acides faibles par exemple des ions pyruvates, des ions lactates et des ions hydrocarbonates sont contenus.

5. Solution selon l'une quelconque des revendications 1 à 4, **caractérisée en ce que** l'acide physiologiquement compatible pour l'acidification à un pH de 3,2 est l'acide chlorhydrique.

6. Solution selon l'une quelconque des revendications 1 à 5, **caractérisée en ce que** la première solution individuelle comporte les constituants qui suivent:
| | |
|---|---|
| Sodium [mmoles/l] | 180-200 |
| Calcium [mmoles/l] | 2-4 |
| Magnésium [mmoles/l] | 0,8-1,2 |
| H⁺ en excès[mmoles/l] | 0,9-1,1 |
| Chlorure [mmoles/l] | 197-210 |
| Glucose [mmoles/l] | 100-500. |

7. Solution selon l'une quelconque des revendications 1 à 6, **caractérisée en ce que** la seconde solution individuelle comporte les constituants qui suivent:
| | |
|---|---|
| Sodium [mmoles/l] | 70-80 |
| Lactate [mmoles/l] | 65-75 |
| Hydrogénocarbonate [mmoles/l] | 4-6 |

8. Solution selon la revendication 6, **caractérisée en ce que** la première solution individuelle comporte les constituants qui suivent:
| | |
|---|---|
| Sodium [mmoles/l] | 193 |
| Calcium [mmoles/l] | 2,5 ou 3,5 |
| Magnésium [mmoles/l] | 1,0 |
| Excès H⁺ [mmoles/l] | 1,0 |
| Chlorure [mmoles/l] | 203 |
| Glucose [mmoles/l] | 166,5, 252 ou 472 |

9. Solution selon la revendication 7, **caractérisée en ce que** la seconde solution individuelle comporte les constituants qui suivent:
| | |
|---|---|
| Sodium [mmoles/l] | 75 |
| Lacatate [mmoles/l] | 70 |
| Hydrogénocarbonate [mmoles/l] | 5,0 |

10. Solution selon l'une quelconque des revendications 1 à 9, **caractérisée en ce que** les deux solutions individuelles peuvent être stockées en étant séparées dans une poche à chambre double.

11. Poche à chambre double contenant une solution selon l'une quelconque des revendications 1 à 10 **caractérisé en ce qu'**il se compose d'une poche en matière synthétique dans laquelle une première chambre avec la première solution et une seconde chambre avec la seconde solution sont agencées côte à côte, les deux chambres étant séparées l'une de l'autre par une soudure qui est dimensionnée de façon qu'elle s'ouvre lors de la pression sur l'une des chambres remplies de liquide pour que le contenu des deux chambres puisse se mélanger.
